# NEUE EUROPÄISCHE PATENTSCHRIFT

(11) **EP 2 196 225 B2**
(45) Veröffentlichungstag und Bekanntmachung des Hinweises auf die Entscheidung über den Einspruch: **30.04.2025**
(45) Hinweis auf die Patenterteilung: 16.10.2019
(21) Anmeldenummer: 09015161.4
(22) Anmeldetag: 08.12.2009
(51) Int. Cl.: A61L 26/00, A61K 9/70

(54) **Wasserlösliche Wirkstoffe in acrylatbasierenden Zubereitungen**
Water-soluble agents in acrylate-based preparations
Matières actives hydrosolubles dans des préparations à base d'acrylate

(30) Priorität: 09.12.2008 DE 102008060904
(43) Veröffentlichungstag der Anmeldung: 16.06.2010
(73) Patentinhaber: Beiersdorf AG, 22529 Hamburg (DE)
(72) Erfinder: Kommolk, Ralf, 03238 Finsterwalte (DE)
(74) Vertreter: Beiersdorf AG

(56) Entgegenhaltungen:
- EP-A2- 0 164 999
- EP-A2- 0 164 999
- WO-A2-2007/025261
- WO-A2-2010/029093
- FR-A1- 2 410 036
- US-B1- 7 198 800
- US-B1- 7 198 800

## Beschreibung

Die Erfindung beschreibt kosmetische Zubereitungen oder Wundversorgungszubereitungen, umfassend ein oder mehrere filmbildende acrylatbasierende Polymere vorzugsweise gelöst in flüchtigen organischen Lösemitteln wie Ethylacetat, Pentan, Hexan und dergleichen, sowie Wasser, wasserlösliche Wirkstoffe und ein oder mehrere Lösevermittler gewählt aus der Gruppe Isopropylalkohol, Propylalkohol, und/oder 2-Phenoxyethanol, wobei als filmbildende Polymere Polymethylacrylat-Isobuten-Monoisopropylmaleat und/oder Poly(methylmethacrylat-butylmethacrylat) gewählt werden.

Bekannte Wundabdeckungen basieren auf filmbildenden Polymerisaten, die mit Hilfe flüchtiger organischer Lösemittel in gelöster Form zur Herstellung von Wundabdeckungen dienen. Die Lösungen können als Aerosole aus einem Aerosolbehälter in dünnen Schichten auf eine zu behandelnde Schnitt-, Schürf- oder Operationswunde aufgesprüht oder durch Pinsel oder anderweitige Auftragsverfahren auf den Wundbereich appliziert werden. Nach Abdunsten des Lösungsmittels bildet sich auf der Wunde ein dünner, zusammenhängender Film aus, der die Verletzungsstelle abdeckt und den Wundbereich gegen äußere Einflüsse schützt.

Flüssige Verbandsmaterialien oder flüssige Pflaster werden solche zusammen mit einem Treibmittel in Aerosolbehältern abgefüllt in den Handel gebracht, den sog. Wundverband-Sprays, aus denen sie bei Bedarf durch Betätigung des Ventilknopfes auf die Wunde aufgesprüht werden können. Bekannt sind Sprühpflastersysteme wie sie in den Patentschriften DE 2343923 und DE 2924042 beschrieben sind.

Beispielsweise Hansaplast^{®} Sprüh-Pflaster besteht im Wesentlichen aus einem filmbildenden Polymer auf Acrylatbasis, gelöst in einem Lösemittelgemisch (Ethylacetat), aus Treibmitteln (n-Pentan, CO2) und einem Zusatzstoff (Menthol) als zusätzlicher Geruchs- und Wirkstoff mit leicht desinfizierender Wirkung.

Die derzeit verwendeten Sprühpflastersysteme weisen eine bakterizide Wirkung im Suspensionsversuch nach DIN EN 1040 nach.

Die bakterizide Wirkung wird durch das verwendete Gemisch aus Menthol mit den flüchtigen organischen Lösemitteln Ethylacetat und Pentan hervorgerufen.

Eine fungizide Wirkung gemäß Suspensionsversuch nach DIN EN 1275 konnte hingegen an den bekannten Sprühpflastersystemen nicht nachgewiesen werden.

Um nicht nur bakteriostatisch sondern auch bakterizid und fungizid zu wirken, müssen den bekannten Sprühpflasterlösung allerdings entsprechende Wirkstoffe zugegeben werden, die sich jedoch aufgrund der unterschiedlichen Polarität nicht oder nur schlecht in den Sprühpflastermischungen lösen.

Aufgabe der vorliegenden Erfindung ist es Sprühpflastersysteme bereit zu stellen, die zusätzlich zur bakteriziden auch eine fungizide Wirkung und eine noch stärkere und schnellere Reduktion der Keimzahlen durch Einsatz geeigneter Wirkstoffe erzielen können.

Menthol, 2-Phenylethanol, Biphenyl-2-ol, 2-Phenoxyethanol, 2-Isopropyl-5-methylphenol (Thymol), 4-Allyl-2-methoxyphenol (Eugenol) sind bekannte Wirkstoffe mit desinfizierender Wirkung. Diese Wirkstoffe bieten den Vorteil, sich problemlos in den vorhandenen Sprühpflastersystemen lösen zu lassen. Sie haben aber auch die Nachteile, erst ab einer relativ hohen Konzentration wirksam zu sein, relativ leichtflüchtig zu sein und zum Teil einen sehr intensiven Geruch zu besitzen.

Aufgabe der vorliegenden Erfindung ist es daher auch ein alternatives Sprühpflastersystem zur Verfügung zu stellen, das insbesondere eine breitere Einsatzmöglichkeit von desinfizierenden wasserlöslichen Wirkstoffen erlaubt.

Antiseptika werden in Wundspüllösungen eingesetzt, die meist auf Wasserbasis unter Verwendung von Polyethylenglykol und Glycerin beruhen. Von Seiten der Wirksamkeit und Verträglichkeit werden als Antiseptika Polyhexanid als das Mittel der Wahl dargestellt, gefolgt von Octenidin in Kombination mit 2-Phenoxyethanol, Chlorhexidin und Benzalkoniumchlorid. Beispiele für Wundspüllösungen sind die Handelsprodukte LAVASEPT, COSMOCIL, OCTENISEPT, PRONTOSAN, EUXYL K600.

Polyhexanid wird als Antiseptikum ebenso in Substanzen, wie Cremes, Salben, Hydrogels, Gele, mit einer höheren Viskosität als Wasser beschrieben (DE 29611266).

DE 102005003262 beschreibt die Verwendung antimikrobieller Polymere als Emulgatoren und Emulgatoren enthaltende antimikrobielle Polymerdispersionen.

In der DE 10212864A1 wird wiederum ein Mischsystem zur Löslichkeitsvermittlung von pharmazeutischen Wirkstoffen in Polymermatrizes beschrieben.

In der Patentschrift US 2004116636A1 werden Polymere beschrieben, in die Biguanide ein polymerisiert sind.

Bekannte empfehlenswerte und speziell für den Einsatzzweck der Schleimhaut- und Wunddesinfektion geeignete Antiseptika, wie beispielsweise Octenidin, Chlorhexidin und Polyhexanid, lassen sich jedoch nicht oder nur schwerlich in den bestehenden Sprühpflastersystem einarbeiten.

Octenidin als Octenidindihydrochlorid ist als quartäre Ammoniumverbindung in den acrylatbasierenden Sprühpflastersystemen nicht löslich.

Auch Chlorhexidin in Form von Chlorhexidindigluconat oder Chlorhexidindiacetat-Monohydrat und Polyhexanid aus der Klasse der Biguanide lassen sich ebenfalls nicht im Lösemittelsystem der bekannten Sprühpflaster lösen.

US 5763412 beschreibt antimikrobielle filmbildende Zubereitungen mit einem flüchtigen Lösemittel und Chlorhexidingluconat. Als Filmbildner werden Cellulosetypen eingesetzt und Chlorhexidingluconat als Weichmacher für Cellulosematerialien verwendet.

DE 2924042 beschreibt klassische Sprühpflastersysteme aus Fumarsäureestern und dessen Herstellung. Lösemittelmischungen u. a. mit Ethylacetat und Alkanen sowie Alkoholen finden Erwähnung. Ebenfalls wir allgemein der Zusatz von blutstillenden und bakteriziden, nicht fungiziden, Additiven beschrieben. Die Möglichkeit des Zusatzes von Wasser und damit auch wasserlöslichen Wirkstoffen findet jedoch keine Erwähnung.

US 4987893 beschreibt polymere Filmbildner auf Basis von Siloxanen.

US 4379863 beschreibt (Meth-) Acrylatpolymerlösungen zur Colostomie-Anwendung. Die Polymere können gelöst in Alkoholen, u.a. in Isopropanol, vorliegen.

US 4584192 beschreibt Acrylatpolymerisate, die immer N-vinyl-lactam enthalten.

US 4310509 und US 7198800 offenbaren klassische Verbandsmaterialien auf Acrylatbasis. Zur Herstellung eines selbstklebenden Films auf einem Träger können u.a. desinfizierende Wirkstoffe mit Lösemittelgemische von Ethylacetat, Wasser und Alkoholen genutzt und Acrylatpolymerisate darin hergestellt bzw. gelöst werden. Als wesentlicher Bestandteil werden 0,1 bis 20 % eines Weichmachers zugesetzt. Sprühbare oder flüssig applizierbare Zubereitungen werden nicht beschrieben.

EP 1150661 offenbart sprühbare filmbildende Zubereitungen, in denen verschiedenste filmbildende Polymer, verschiedenste Wirkstoffe sowie Lösevermittler und Permeationsverbesserer aufgeführt werden. In einer Reihe möglicher Polymere sind u.a. acrylatbasierende Polymere genannt. In einer Reihe möglicher Wirkstoffe sind auch hydrophile Wirkstoffe mit aufgeführt. In einer Reihe möglicher Lösemittel ist u.a. Wasser und Isopropylalkohol genannt. Zwingender Bestandteil zur Erreichung der Filmbildung ist der Weichmacher Dimethylisosorbid

WO 03063923 offenbart eine sprühfähige Wundzubereitung, die auf der Haut ein Gel bildet. In einer Reihe möglicher Polymere sind u.a. hydrophile Makromere, wie u.a. acryltbasierende Polymere genannt. Die hier beschriebenen hydrophilen Hydroxy-Polyacrylate sind durchweg polar und können somit leichter in Wasser gelöst oder gequollen werden. Als weitere optionale Möglichkeit eines Lösemittels wird der Zusatz an Wasser sowie Isopropanol genannt.

US 20040247655 offenbart klassische Klebmassen. Diese können auf Acrylatpolymeren basieren. Die Polymere umfassen zwangsweise quervernetztes Poly (N-Vinyl lactam) und ein Quellagens, insbesondere Di- oder Triglycerol. Als weitere Quellmittel werden darin neben anderen bekannten Quellmitteln u.a. Wasser und Isopropanol genannt.

EP 0 164 999 A2 offenbart filmbildende acrylatbasierende Zubereitungen, die antimikrobielle Wirkstoffe insbesondere Iod, umfassen. In anderen Ausführungsformen werden auch Chlorhexidin als Wirkstoff oder Isopropylalkohol, Ethanol oder Aceton als Lösemittel beschrieben, wobei Aceton bevorzugt wird.

Problematisch bei vielen dieser Polymersysteme ist, dass entweder die Polymere selber hydrophob gestaltet sind und/oder hydrophobe Lösemittelgemische, wie Ethylacetat und Alkane, wie Treibgasen Hexan, Propan, Butan, umfassen. In diesen.hydrophoben Systemen ist ein wirkungsvolles Einarbeiten hydrophiler Wirkstoffe und Wasser nicht möglich.

Aufgabe der vorliegenden Erfindung ist es wasserlösliche Wirkstoffe, insbesondere wasserlösliche Antiseptika, sowie Wasser selber als für diese Wirkstoffe notwendiges Lösemittel in hydrophobes acrylatbasierende Filmpflastersystem einarbeiten zu können. Das Filmpflastersystem umfasst dabei klassische organische Lösemittel wie Ethylacetat und/oder Alkane, welche hauptsächlich flüchtig sind und nicht Wasser oder wasserlösliche Wirkstoffe aufnehmend wirken.

Neben der Wundbehandlung sind auch allgemein kosmetische Behandlungen und Hautpflegeprodukte mittels wasserlöslicher Wirkstoffe von den Verbrauchern gewünscht. Auch hier ergibt sich das Problem, dass wasserlösliche Wirkstoffe in hydrophobe acrylatbasierende Applikationssysteme nicht oder nur schwerlich einarbeitbar bzw. langfristig nicht stabil hergestellt werden können.

Auch bei dieser Problemstellung ist in Analogie zu der oben beschriebenen Wundbehandlung zu beachten, dass auf dem zu behandelnden Hautareal ein flächiger Film erhalten wird, der einerseits dazu dient, das entsprechende Hautareal möglichst wenig sichtbar abzudecken, andererseits sollen wasserlösliche Wirkstoffe zu positiven Beeinflussung von kosmetisch nicht erwünschten Hauterscheinungen an und in die Haut abgegeben werden.

Hierzu zählen sowohl lokal begrenzte Hauterscheinungen wie entzündliche Reaktionen (z.B. Pickel, Insektenstiche, eingewachsene (Bart-) Haare etc.) und Hyper- sowie Hypopigmentierungen (Altersflecken, Melsma, Sommersprossen, postinflammatorische Hyperpigmentierungen jeglicher Art beispielsweise in Folge von einer *pseudofollikolitis barbae,* Vitiligo), Falten, Narben, Augenringe, Dehnungsstreifen (beispielsweise in Folge von Schwangerschaften oder Körperwachstum oder Gewichtszunahme) wie auch unerwünschte flächige Hauterscheinungen wie beispielsweise Cellulite, insgesamt ungleichmäßig pigmentierte Haut (*uneven skin tone*).

Die oben genannten unerwünschten Hauterscheinungen sollen beispielhaft die wichtigsten nennen, für die eine einfache Behandlungsform gesucht wird.

Gelöst werden die Aufgaben durch eine Zubereitung wie sie in den Hauptansprüchen dargelegt ist. Die Unteransprüche geben bevorzugte Ausführungsformen der kosmetischen oder Wundversorgungszubereitungen wieder.

Die Erfindung umfasst eine Zubereitung zur Wundheilung oder -pflege, umfassend ein oder mehrere filmbildende hydrophobe acrylatbasierende Polymere, Wasser, wasserlösliche Wirkstoffe und ein oder mehrere Lösevermittler gewählt aus der Gruppe Isopropylalkohol, Propylalkohol, und/oder 2-Phenoxyethanol, wobei als filmbildende Polymere Polymethylacrylat-Isobuten-Monoisopropylmaleat und/oder Poly(methylmethacrylat-butylmethacrylat) gewählt werden.

Die Zubereitungen umfassen des Weiteren vorteilhaft flüchtige organische Lösemittel, insbesondere gewählt aus Ethylacetat und/oder unpolaren Treibgasen, wie Alkane.

Mit der Erfindung wird erstmals die Möglichkeit eröffnet, in lösemittelhaltige acrylatbasierende Polymere, wie sie beispielsweise in bekannten Sprühpflastern eingesetzt werden, durch Verwendung von Lösevermittlern, bevorzugt Isopropanol, Wasser einzubringen bzw. zu lösen und dadurch wasserlösliche Wirkstoffe (z. B Antiseptika, wässrige Auszüge/Extrakte) in gelöster Form in das sprühfähige Produkt einzubringen, ohne dass dessen Filmbildungseigenschaften merklich beeinflusst werden.

Als filmbildende Polymere werden Polymethylacrylat-Isobuten-Monoisopropylmaleat und/oder Poly(methylmethacrylat-butylmethacrylat) gewählt.

Die erfindungsgemäßen Acrylatpolymere sind hydrophob und nicht in Wasser quellbar. Sie unterscheiden sich daher von den im Stand der Technik erwähnten Acrylatpolymeren. Zu diesen Polymeren wird Wasser als Löse- bzw. Quellmittel zugesetzt.

Das erfindungsgemäße System umfasst bevorzugt organische, und damit hydrophobe, flüchtige Lösemittel, wie Ethylacetat oder Alkane, und bildet in Kombination mit den hydrophoben Acrylaten das hydrophobe System, in das wasserlösliche Wirkstoffe und Wasser nur mit Hilfe der erfindungsgemäßen Lösevermittler eingearbeitet werden können.

In keinem der Dokumente des Standes der Technik, wie er zuvor dargestellt wurde, ist die erfindungsgemäße Kombination aus hydrophoben acrylatbasierenden Polymeren, Wasser, insbesondere zu einem Anteil von mehr als 4 Gew.%, wasserlösliche Wirkstoffe und Isopropylalkohol, Propylalkohol, und/oder 2-Phenoxyethanol offenbart.

Insbesondere kann in den erfindungsgemäßen Zubereitungen auf den Zusatz von Quellmitteln insbesondere Di- oder Triglycerol, verzichtet werden. Auch Polymerzusätze oder besondere Ausgestaltungen, wie Poly (N-Vinyl lactam), sind erfindungsgemäß nicht notwendig.

Die erfindungsgemäßen Zubereitungen umfassen einfach aufgebaute, bekannte Acrylatpolymersysteme, die durch die erfindungsgemäßen Lösevermittler und Wasser mit hydrophilen Wirkstoffen erstmals unproblematisch beladen werden können. Kostenträchtige Neuformulierungen und Anpassungen sowie der Zusatz weiterer Chemikalien entfällt. Wasser ist in der Zubereitung bevorzugt zu einem Anteil von mehr als 4 Gew%, bezogen auf die Gesamtmasse der Zubereitung enthalten. Insbesondere beträgt der Anteil an Wasser mehr als 12 Gew.%.

Als wasserlösliche Wirkstoffe können bevorzugt kosmetisch wirksame Stoffe im erfindungsgemäßen System verwendet werden.

Vorteilhaft können folgende kosmetische Stoffe als wasserlösliche Wirkstoffe gewählt werden:
Vitamin C und seine Derivate insbesondere Ascorbylpalmitat, Na- und Mg-Ascorbylphosphat und Ascorbylacetat, Folsäure und ihre Derivate (insbesondere Tetrahydrofolsäure und Dihydrofolsäure), Niacin und seine Derivate (insbesondere Niacinamid), Kreatin und Kreatinin sowie die jeweiligen Derivate, alpha-Glycosyl-Rutin sowie seine Derviate, verschiedene Extrakte der Süßholzwurzel, Licochalcone, insbesondere das Licochalocon-A, 4-Butyl-Resorcinol, Coffein, Glyceryl-Glucose, Glycerin, Hyaluronsäure, Hydroxy-Prolin, Ac-N-Hydroxy-Prolin, Carnitin, Isoflavon, Soja- und Klee-Extrakte, Grüntee-Extrakte, Eucalyptusöl, Harnstoff und Mineralsalze (wie z. B. NaCl, Meeresmineralien) sowie Osmolyte (wie z. B. Taurine, Inositol, Betain, quartäre Ammoniumverbindungen), Silymarin, Dexpanthenol, Inhibitoren des Prostaglandinstoffwechsels, insbesondere der Cyclooxygenase und des Leukotrienstoffwechsels, wie z.B. Acetylsalicylsäure. Des Weiteren können Polidocanol, Hamamelis Extrakt, Magnolienextrakt und Pfingstrosenextrakt eingesetzt werden.

Hierbei eignet sich in erfindungsgemäßen Formulierungen 4-Butyl-Resorcinol ganz besonders aufgrund der positiven kosmetischen Beeinflussung bei lokalen Hyperpigmentierungen.

Alpha-Glycosylrutin sowie Licochalocone-A erweisen vorteilhaft bei lokal begrenzten entzündlichen Reaktionen. Carnitin und Coffein sind in den erfindungsgemäßen Formulierungen besonders gut geeignet zur positiven Beeinflussung bei der Behandlung von Cellulite. Zur positiven kosmetischen Beeinflussung von Falten sind Folsäure, Kreatin/ Kreatinin und Vitamin C sowie ihre Derivate sowie natürliche und synthetische Flavonoide besonders gut geeignet.

Vorteilhaft ist auch der Zusatz an Glyceryl-Glucose.

Für medizinische Anwendungen, dem bekannten Einsatzgebiet filmbildender acrylatbasierender Polymerzubereitungen in einem organischen flüchtigen Lösemittel, können erfindungsgemäßen Zubereitungen wundpflegende und/oder wundheilungsfördernde Wirkstoffe wie Polidocanol, Hamamelis Extrakt, Dexpanthenol o.ä. zugesetzt werden.

Weitere wasserlösliche Wirkstoffe, die speziell für den Einsatzzweck der Schleimhaut- und Wunddesinfektion geeignete Antiseptika, insbesondere Benzalkoniumchlorid, Octenidin (Octenidindihydrochlorid), Chlorhexidin, Chlorhexidindigluconat, Chlorhexidindiacetat-Monohydrat und/oder Polyhexanid können bevorzugt in das erfindungsgemäße Kit eingesetzt werden.

Die Löslichkeit dieser Stoffe wurde in bekannten Sprühpflastersystem (Hansaplast Sprühpflaster) im Vergleich zum erfindungsgemäßen System getestet.

Dabei wurde festgestellt, dass sich die wasserlöslichen Wirkstoffe nur im erfindungsgemäßen Kit (System) anwendungsgemäß einbringen lassen können.

Aus der Gruppe der quartären Ammoniumverbindungen lässt sich Benzalkoniumchlorid sowohl in bekannte als auch in das erfindungsgemäße acrylatbasierende Sprühpflastersystem einarbeiten.

Octenidin als Octenidindihydrochlorid als eine weitere quartäre Ammoniumverbindung ist wiederum in den Sprühpflastersystemen des Standes der Technik nicht löslich. Auch der Zusatz von Isopropanol, in dem sich Octenidindihydrochlorid leicht lösen lässt, brachte keine Verbesserung. In Isopropanol gelöstes Octenidindihydrochlorid fällt wieder aus, wenn man es in das acrylatbasierende Sprühpflastersystem einbringt.

Chlorhexidin in Form von Chlorhexidindigluconat oder Chlorhexidindiacetat-Monohydrat und Polyhexanid aus der Klasse der Biguanide lassen sich ebenfalls nicht im Lösemittelsystem für Sprühpflaster lösen. Nur in Wasser sind sie vollständig (unbegrenzt), in Glycerin, Triethylenglykol, Polyethylenglykolen sind sie nur teilweise (begrenzt) löslich. Gibt man derartige Wirkstofflösungen in das Sprühpflastersystem, so fallen die Wirkstoffe aus und/oder es trennt sich die wässrige von der organischen Phase. In dem Sprühpflastersystem des Standes der Technik ist Wasser nicht löslich.

Überraschenderweise ist es nun gelungen, durch Zusatz eines Lösevermittlers wie Isopropylalkohol, Propylalkohol und/oder 2-Phenoxyethanol und Wasser in das Sprühpflastersystem zu bringen, in dem sich die wasserlöslichen Wirkstoffe (z. B. Octenidin, Chlorhexidin, Polyhexanid) wiederum lösen lassen. Völlig unerwartet erhält man durch Zugabe des Lösevermittlers stabile Lösungen/Mischungen aus denen weder das Polymer, noch die Wirkstoffe ausfallen. Es trennt sich auch nicht die wässrige von der organischen Phase.

Vorteilhaft sind durch diesen Zusatz an Lösevermittler, Wasser und wasserlöslichen Wirkstoffen auch die filmbildenden Eigenschaften des Wundversorgungskits unverändert geblieben.

Dies haben die oben genannten Untersuchungen als auch einfache Tests durch Auftragen auf den Handrücken und Tragen über 24 h ergeben.

Die Anteile an den wasserlöslichen Wirkstoffen beträgt allgemein vorteilhaft weniger als 1 Gew.-%, bevorzugt im Bereich von 0,01 bis 0,5 Gew.-%, bezogen auf die Gesamtmasse der Zubereitung. Höhere Anteile als 0,1 % an Polyhexanid in der Gesamtmischung scheinen durch die eingesetzte Lösemittelkombinationen optimal zu sein, was eine ausreichende Wirkstoffkonzentration darstellt.

Bei einzelnen Wirkstoffen, wie beispielsweise Glyceryl-Glucose, ist der Anteil bis zu 15 Gew.% zu wählen, vorteilhaft im Bereich von etwa 9 bis 14 Gew.%, besonders bevorzugt im Bereich von 10 bis 12 Gew.%, bezogen auf die Gesamtmasse der Zubereitung.

Vorteilhaft ist weiterhin, dass die erfindungsgemäße Zubereitung lediglich die erwähnten Inhaltsstoffe umfasst und so auf weitere Stoffe verzichtet werden kann.

Bevorzugt umfasst die erfindungsgemäße Zubereitung nur
- Polymethylacrylat-Isobuten-Monoisopropylmaleat und/oder Poly(methylmethacrylat-butylmethacrylat)
- ein oder mehrere wasserlösliche Wirkstoffe
- ein oder mehrere Lösevermittler gewählt aus der Gruppe Isopropylalkohol, Propylalkohol, und/oder 2-Phenoxyethanol
- Wasser
- ggf. organische flüchtige Lösemittel, wie Ethylacetat und/oder Treibgase, insbesondere flüchtige Alkane.

Bevorzugt sind sowohl Ethylacetat als auch Kohlenwasserstofftreibgase enthalten.

Die Verwendung der Zubereitung ist bevorzugt zur kosmetischen Pflege oder kosmetischen Behandlung der Haut oder Haare geeignet, aufgrund der handhabungsfreundlichen Sprüh- bzw. Pinselapplikationsmöglichkeit.

Erfindungsgemäße Anwendungsgebiete der Zubereitungen, als Pinsel- oder Sprühapplikation, sind bevorzugt die Behandlung lokal begrenzter Hauterscheinungen wie entzündliche Reaktionen (z.B. Pickel, Insektenstiche, eingewachsene (Bart-) Haare etc.) und Hyper- sowie Hypopigmentierungen (Altersflecken, Melsma, Sommersprossen, postinflammatorische Hyperpigmentierungen jeglicher Art beispielsweise in Folge von einer *pseudofollikolitis barbae,* Vitiligo), Falten, Narben, Augenringe, Dehnungsstreifen (beispielsweise in Folge von Schwangerschaften oder Körperwachstum oder Gewichtszunahme) wie auch unerwünschte flächige Hauterscheinungen wie beispielsweise Cellulite, insgesamt ungleichmäßig pigmentierte Haut (*uneven skin tone*).

Bevorzugt wird die erfindungsgemäße Zubereitung in der Wundbehandlung, beispielsweise als Wundspray, angewendet. Entsprechend werden dann Wundheilungsfördernde hydrophile Wirkstoffe gewählt.

Gleichermaßen ist die kosmetische Anwendung, beispielsweise bei der Cellulitebehandlung vorteilhaft.

Bevorzugt ist das erfindungsgemäße Wundversorgungskit als Sprühpflaster formuliert, so dass neben den erfindungsgemäßen Bestandteilen noch weitere Stoffe, wie Treibgase oder Parfüme, zugegen sein können.

Als Aerosol, in Quetschflaschen oder in Treibgasflaschen ist eine Anwendung möglich und bevorzugt.

Bevorzugte Applikationsform der erfindungsgemäßen Zubereitung ist die Aerosolform. Flüchtige organische Verbindungen (Abk.: VOC bzw. VOCs nach volatile organic compound[s]) sind organische, also kohlenstoffhaltige Stoffe, die leicht verdampfen (flüchtig sind) bzw. schon als Gas bei niedrigen Temperaturen (z. B. Raumtemperatur) vorliegen.

Als Treibgase werden erfindungsgemäß bevorzugt Propan, Propen, n-Butan, iso-Butan, isoButen, n-Pentan, Penten, iso-Pentan, iso-Penten, Hexan, Methan, Ethan, Dimethylether, Stickstoff, Luft, Sauerstoff, Stickstoffoxide, Lachgas, 1,1,1,3 Tetrafluorethan, Heptafluoro-n-propan, Perfluorethan, Monochlordifluormethan, 1,1-Difluorethan einzeln oder in Kombination eingesetzt. Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuss vorliegt. Besonders bevorzugt sind Propan, Butan, iso-Butan bzw. Mischungen dieser Treibgase.

Die genannten Gase können im Sinne der vorliegenden Erfindung jeweils einzeln oder in beliebigen Mischungen zueinander verwendet werden.

Als Druckgasbehälter kommen bekannte Gefäße aus Metall (Aluminium, Weißblech), geschütztem bzw. nicht-splitterndem und splitterndem Glas oder Kunststoff in Frage, bei deren Auswahl Druck- und Bruchfestigkeit, Korrosionsbeständigkeit, leichte Füllbarkeit als auch ästhetische Gesichtspunkte, Handlichkeit, Bedruckbarkeit etc. eine Rolle spielen. Auch eine Pinselapplikation des erfindungsgemäßen Kits ist erfindungsgemäß. Diese kann dann anlog wie sie von Nagellacken bekannt ist erfolgen, wobei der Pinsel im Deckel des Behältnisses angebracht ist. Desweiteren kann die Applikation mittels eines Stiftes erfolgen. Hier ist zum einen eine Applikation über einen Filzstift zu nennen sowie die Applikation über einen Roller-Pen (analog zu einer Roll-on-Applikation).

Nachfolgende Beispiele illustrieren einige erfindungsgemäße Wundversorgungszubereitungen. Die angegebenen Anteile sind Gewichtsanteile bezogen auf die Gesamtmasse der Zubereitungen.

Den beispielhaft dargestellten Lösungen können als Aerosolapplikation Treibgase zugesetzt werden.

### Beispiele

| | sprühfähige Lösungen | | |
|---|---|---|---|
| | Lsg.1 | Lsg.2 | Lsg.3 |
| Ethylacetat | 65.42% | 64.87% | 64.86% |
| Menthol | 0.52% | 0.49% | 0.45% |
| Pentan | 6.16% | 6.27% | 5.86% |
| Poly(methylacrylat-Isobuten-Monoisopropylmaleat) | 8.83% | 8.71% | 8.91% |
| Triethylenglykol | 1.12% | 1.22% | 1.33% |
| Octenidindihydrochlorid | 0.01% | 0.09% | 0.19% |
| 2-Phenoxyethanol | 0.11% | 0.88% | 0.90% |
| Isopropylalkohol | 13.41% | 13.10% | 13.12% |
| Wasser | 4.41% | 4.37% | 4.37% |
| | 100.00% | 100.00% | 100.00% |

| | sprühfähige Lösung | |
|---|---|---|
| | Lsg. 4 | Lsg.5 |
| Ethylacetat | 58.11% | 57.81 % |
| Poly(methylacrylat-Isobuten-Monoisopropylmaleat) | 9.70% | 9.26% |
| Chlorhexidindiacetat-Monohydrat | 0.21% | 0.46% |
| Isopropylalkohol | 19.26% | 19.00% |
| Wasser | 12.72% | 13.48% |
| | 100.00% | 100.00% |

| | sprühfähige Lösung | |
|---|---|---|
| | Lsg. 6 | Lsg. 7 |
| Ethylacetat | 54.86% | 64.86% |
| Poly(methylmethacrylat-butylmethacrylat) | 9.01% | 9.01% |
| 2-Phenoxyethanol | 2.22% | 12.22% |
| Polyhexanid | 0.10% | 0.10% |
| Isopropylalkohol | 20.75% | - |
| Wasser | 13.05% | 13.80% |
| | 100.00% | 100.00% |

| | sprühfähige Lösung | |
|---|---|---|
| | Lsg. 8 | Lsg.9 |
| Ethylacetat | 58.11% | 57.81% |
| Poly(methylacrylat-Isobuten-Monoisopropylmaleat) | 9.70% | 9.26% |
| 4-Butyl-Resorcionol | 0.31% | 0.40% |
| Isopropylalkohol | 19.26% | 19.00% |
| Wasser | 12.62% | 13.54% |
| | 100.00% | 100.00% |

| | sprühfähige Lösung | |
|---|---|---|
| | Lsg. 10 | Lsg. 11 |
| Ethylacetat | 58.11% | 57.81% |
| Poly(methylacrylat-Isobuten-Monoisopropylmaleat) | 9.70% | 9.26% |
| Licochalcone A | 0.10% | 0.30% |
| Isopropylalkohol | 19.26% | 19.00% |
| Wasser | 12.83% | 13.64% |
| | 100.00% | 100.00% |

## Patentansprüche

1. Kosmetische Zubereitung oder Wundversorgungszubereitung umfassend ein oder mehrere filmbildende hydrophobe acrylatbasierende Polymere, Wasser, wasserlösliche Wirkstoffe und ein oder mehrere Lösevermittler gewählt aus der Gruppe Isopropylalkohol, Propylalkohol, und/oder 2-Phenoxyethanol, **dadurch gekennzeichnet, dass** als Polymere Poly(methylacrylat-Isobuten-Monoisopropylmaleat) oder Poly(methylmethacrylat-butylmethacrylat) gewählt werden.

2. Zubereitung nach Anspruch 1 **dadurch gekennzeichnet, dass** ein oder mehrere Wirkstoffe gewählt werden aus der Gruppe 4-Butyl-Resorcinol, Alpha-Glycosylrutin, Licochalocone-A, Carnitin, Coffein, Folsäure, Kreatin/ Kreatinin, Vitamin C, natürliche und synthetische Flavonoide und Glyceryl-Glucose.

3. Zubereitung nach Anspruch 1 **dadurch gekennzeichnet, dass** ein oder mehrere Wirkstoffe gewählt werden aus der Gruppe der Antiseptika, insbesondere Benzalkoniumchlorid, Octenidin (Octenidindihydrochlorid), Chlorhexidin, Chlorhexidindigluconat, Chlorhexidindiacetat-Monohydrat und/oder Polyhexanid

4. Zubereitung nach Anspruch 1 **dadurch gekennzeichnet, dass** ein oder mehrere Wirkstoffe gewählt werden aus der Gruppe Dexpanthenol, Polidocanol, Hamamelis Extrakt, Magnolienextrakt und Pfingstrosenextrakt.

5. Zubereitung nach einem der vorstehenden Ansprüche **dadurch gekennzeichnet, dass** Wasser zu einem Anteil von mehr als 4 Gew.%, insbesondere mehr als 12 Gew.%, bezogen auf die Gesamtmasse der Zubereitung, enthalten ist.

6. Zubereitung nach einem der vorstehenden Ansprüche **dadurch gekennzeichnet, dass** ein oder mehrere flüchtige organische Lösemittel enthalten sind.

7. Zubereitung nach Anspruch 7 **dadurch gekennzeichnet, dass** Ethylacetat und unpolare Treibgase, wie Alkane, enthalten sind.

8. Zubereitung nach einem der vorstehenden Ansprüche **dadurch gekennzeichnet, dass** keine Quellmitteln enthalten sind.

9. Verwendung der Zubereitung nach einem der Ansprüche 1 bis 8 zur Herstellung einer Wundversorgungszubereitung.

10. Verwendung der Zubereitung nach einem der Ansprüche 1 bis 8 zur kosmetischen Pflege oder kosmetischen Behandlung der Haut oder Haare.

## Claims

1. Cosmetic preparation or wound care preparation comprising one or more film-forming hydrophobic acrylate-based polymers, water, water-soluble active ingredients and one or more solubilizers selected from the group comprising isopropyl alcohol, propyl alcohol and/or 2-phenoxyethanol, **characterized in that** the polymers selected are poly(methyl acrylate-isobutene-monoisopropyl maleate) or poly(methyl methacrylate-butyl methacrylate).

2. Preparation according to Claim 1, **characterized in that** one or more active ingredients are selected from the group comprising 4-butylresorcinol, alpha-glucosylrutin, licochalcone A, carnitine, caffeine, folic acid, creatine/creatinine, vitamin C, natural and synthetic flavonoids and glyceryl glucose.

3. Preparation according to Claim 1, **characterized in that** one or more active ingredients are selected from the group of antiseptics, especially benzalkonium chloride, octenidine (octenidine dihydrochloride), chlorhexidine, chlorhexidine digluconate, chlorhexidine diacetate monohydrate and/or polyhexanide.

4. Preparation according to Claim 1, **characterized in that** one or more active ingredients are selected from the group comprising dexpanthenol, polidocanol, Hamamelis extract, magnolia extract and peony extract.

5. Preparation according to any of the preceding claims, **characterized in that** water is present at a proportion of more than 4% by weight, especially more than 12% by weight, based on the total mass of the preparation.

6. Preparation according to any of the preceding claims, **characterized in that** one or more volatile organic solvents are present.

7. Preparation according to Claim 7, **characterized in that** ethyl acetate and non-polar propellant gases, such as alkanes, are present.

8. Preparation according to any of the preceding claims, **characterized in that** no swelling agents are present.

9. Use of the preparation according to any of Claims 1 to 8 for producing a wound care preparation.

10. Use of the preparation according to any of Claims 1 to 8 for cosmetic care or cosmetic treatment of skin or hair.

## Revendications

1. Préparation cosmétique ou préparation pour le soin des plaies comprenant un ou plusieurs polymères hydrophobes filmogènes à base d'acrylate, de l'eau, des agents actifs solubles dans l'eau et un ou plusieurs solubilisants choisis dans le groupe de l'alcool isopropylique, l'alcool propylique et/ou le 2-phénoxyéthanol, **caractérisée en ce qu'**un poly(acrylate de méthyle-isobutène-maléate de monoisopropyle) ou un poly(méthacrylate de méthyle-méthacrylate de butyle) sont choisis en tant que polymères.

2. Préparation selon la revendication 1 **caractérisée en ce qu'**un ou plusieurs agents actifs sont choisis dans le groupe du 4-butyl-résorcinol, l'alpha-glycosylrutine, la licochalcone A, la carnitine, la caféine, l'acide folique, la créatine/créatinine, la vitamine C, les flavonoïdes naturels et synthétiques et le glycéryl-glucose.

3. Préparation selon la revendication 1 **caractérisée en ce qu'**un ou plusieurs agents actifs sont choisis dans le groupe des antiseptiques, notamment le chlorure de benzalkonium, l'octénidine (dichlorhydrate d'octénidine), la chlorohexidine, le digluconate de chlorhedixine, le monohydrate de diacétate de chlorhexidine et/ou le polyhexanide.

4. Préparation selon la revendication 1 **caractérisée en ce qu'**un ou plusieurs agents actifs sont choisis dans le groupe du dexpanthénol, le polidocanol, l'extrait d'hamamélis, l'extrait de magnolia et l'extrait de pivoine.

5. Préparation selon l'une quelconque des revendications précédentes, **caractérisée en ce que** l'eau est contenue en une proportion de plus de 4 % en poids, notamment de plus de 12 % en poids, par rapport à la masse totale de la préparation.

6. Préparation selon l'une quelconque des revendications précédentes, **caractérisée en ce qu'**un ou plusieurs solvants organiques volatils sont contenus.

7. Préparation selon la revendication 7, **caractérisée en ce que** de l'acétate d'éthyle et des gaz propulseurs apolaires, tels que des alcanes, sont contenus.

8. Préparation selon l'une quelconque des revendications précédentes, **caractérisée en ce qu'**aucun agent gonflant n'est contenu.

9. Utilisation de la préparation selon l'une quelconque des revendications 1 à 8 pour la fabrication d'une préparation pour le soin des plaies.

10. Utilisation de la préparation selon l'une quelconque des revendications 1 à 8 pour le soin cosmétique ou le traitement cosmétique de la peau ou des cheveux.
